# EUROPÄISCHE PATENTSCHRIFT

(11) **EP 2 394 740 B1**
(45) Veröffentlichungstag und Bekanntmachung des Hinweises auf die Patenterteilung: **02.01.2013**
(21) Anmeldenummer: 10005952.6
(22) Anmeldetag: 09.06.2010
(51) Int. Cl.: B01L 3/00, B01L 3/02, G01N 15/14, B01L 99/00, C12M 1/00

(54) **Vorrichtung zur Dosierung von Zellen, Verfahren zur Dosierung von Zellen sowie Verwendung der Vorrichtung**
Device and method for metering and dispensing of cells and use of the device
Couvert pour la manipulation d'aliments et récipient pour aliments pour le transport et le stockage d'aliments doté d'un couvert amovible

(43) Veröffentlichungstag der Anmeldung: 14.12.2011
(73) Patentinhaber: KIST-Europe Forschungsgesellschaft mbH, 66123 Saarbrücken (DE)
(72) Erfinder: Jungtae, Kim Dr.-Ing., D-66121 Saarbrücken (DE); Krause, Holger Dipl.-Ing, D-66540 Neukirchen (DE); Park, Jihwang M.Sc, D-66125 Saarbrücken (DE); Beck, Sebastian Dipl.-Ing, D-66386 St. Ingbert (DE)
(74) Vertreter: Pfenning, Meinig & Partner GbR

(56) Entgegenhaltungen:
- EP-A2- 0 177 718
- EP-A2- 1 533 605
- US-A1- 2007 269 348
- US-A1- 2008 286 751
- ANDERSSON H ET AL: "Microfluidic devices for cellomics: a review", SENSORS AND ACTUATORS B, ELSEVIER SEQUOIA S.A., LAUSANNE, CH, Bd. 92, Nr. 3, 15. Juli 2003 (2003-07-15), Seiten 315-325, XP004427497, ISSN: 0925-4005, DOI: DOI:10.1016/S0925-4005(03)00266-1
- CHEN C C ET AL: "Micromachined bubble-jet cell sorter with multiple operation modes", SENSORS AND ACTUATORS B, ELSEVIER SEQUOIA S.A., LAUSANNE, CH, Bd. 117, Nr. 2, 12. Oktober 2006 (2006-10-12), Seiten 523-529, XP025112319, ISSN: 0925-4005, DOI: DOI:10.1016/J.SNB.2006.05.011 [gefunden am 2006-10-12]

## Beschreibung

Die Erfindung betrifft eine Vorrichtung zur Dosierung von Zellen sowie ein Verfahren zur Dosierung von Zellen. Weiterhin betrifft die Erfindung die Verwendung der Vorrichtung zur Zelldosierung.

Es ist bekannt, dass durch Roboter-unterstützte Dosiersysteme mehr als 10.000 Screenings pro Tag möglich sind. Um die Kosten für diese Tests gering zu halten, wird zum einen die Minimierung des Probenvolumens angestrebt, zum anderen werden die Prüfungsverfahren optimiert. Genaue Dosierungen im Sub-Mikroliter-Bereich sind dabei eine wichtige Anforderung an konventionelle Dosiergeräte für HTS-Anwendungen (High Througput Screening).

In der Vergangenheit wurden sog. cell-based assays (Prüfung von Medien, die Zellen enthalten) in der Medikamentenforschung und Biowissenschaft hauptsächlich manuell oder mit Hilfe einfacher Geräte durchgerührt, da die Anwendung eines HTS-Systems für Zellen schwierig ist. Dabei sind das exakte Volumen und die genaue Anzahl der zelle in den Proben wichtig. Ein konventionelles HTS-System kann jedoch die Anzahl der Zellen nicht kontrollieren, sondern nur das Volumen. Mit weiterer Entwicklung der zellbasierten Versuchsreihen wird aktuell jedoch ein geeignetes HTS-System benötigt. Es sind bereits Auswertungsansätze für zellbasierte Versuchsreihen mit 100 Zellen bis hin zu nur einer Zelle auf dem Markt.

Konventionelle HTS-Systeme kontrollieren nur das Volumen der Probe. Das Ergebnis der Auswertung ist jedoch stark von der Anzahl der Zellen in der Probe abhängig, wobei diese Abhängigkeit mit sinkender Zellzahl in der Probe steigt.

Aus der US 2008/0286751 A1 ist eine Vorrichtung bekannt, mit der Tropfen dosiert werden können. Diese Vorrichtung ist aus einem ersten Kanal, einem zweiten Kanal, einem Schnittpunkt dieser Kanäle sowie einer Öffnung, an der der Tropfen austritt, versehen.

Andersson & van den Berg ("Microfluidic devices for cellomics: a review", Sensors and Acuators B, Bd. 92, Nr. 3 (2003), S. 315-325) offenbaren mikrofluidische Vorrichtungen, welche auf einem elektrischen und meschanischen Prinzip basieren und beispielsweise in der Probenentnahme und Sortierung von Zellen verwendet werden. Ferner werden auch bekannte Vorrichtungen zur Zellsortierung und Zellbehandlung sowie Flow-Cytometer vorgestellt. Abschließend werden eine Reihe von Lab-on-Chip (LOC) Vorrichtungen offenbart, welche für Zellstudien herangezogen werden.

Bei konventionellen Systemen liegt die Fehlerwahrscheinlichkeit bei dem Dosieren von Volumen bei weniger als 2 %, die dazugehörige Fehlerwahrscheinlichkeit für die Zellanzahl dagegen bei über 10 %. Darüber hinaus ist bei Proben, die nur eine Zelle enthalten, die genaue Kontrolle der Zellzahl essentiell.

Ausgehend hiervon ist es Aufgabe der vorliegenden Erfindung, ein Dosieren von Zellen mit geringer Fehlerwahrscheinlichkeit sowohl in Bezug auf das Volumen des Tropfens als auch in Bezug auf die Zellzahl im Tropfen zu ermöglichen.

Diese Aufgabe wird durch die Vorrichtung mit den Merkmalen des Anspruchs 1 gelöst. Anspruch 13 betrifft ein Verfahren zur Dosierung von Zellen und Anspruch 20 die Verwendung der Vorrichtung. Weitere vorteilhafte Ausführungsformen sind in den abhängigen Ansprüchen enthalten.

Die erfindungsgemäße Vorrichtung zur Dosierung von Zellen besteht aus einem Grundkörper, wobei in mindestens einer Ebene des Grundkörpers mindestens ein Einlass- und Auslasskanal für die Trägerflüssigkeit, sowie mindestens ein Reinigungskanal und im Wesentlichen senkrecht zu den in dieser Ebene angeordneten Kanälen mindestens ein weiterer Einlasskanal für das Dosiermedium vorgesehen ist und diese Kanäle in ein Zellsammlermodul münden, wobei die Kanäle mindestens jeweils ein Ventil und/oder eine Diffusorstruktur aufweisen, wobei das Zellsammlermodul einen Zellauslass mit einem Dosierventil aufweist und wobei eine Steuerung für die Auslösung des Dosiervorgangs und eine Zellerkennungsvorrichtung vorgesehen sind, wobei die Zellerkennungsvorrichtung auf oder am Grundkörper angeordnet ist oder im Grundkörper integriert ist.

Durch die erfindungsgemäße Vorrichtung ist eine Dosierung von einer kontrollierten Zellzahl in einem Tropfen mit bestimmtem Volumen möglich. Weiterhin enthält dieser Tropfen kaum Verunreinigungen bzw. ungewünschte Moleküle, da durch die erfindungsgemäße Vorrichtung eine Rückspülfunktion eine Reinigung des Zellsammlers ermöglicht wird. Darüber hinaus ist die erfindungsgemäße Vorrichtung wenig anfällig für Konzentrationsschwankungen der Zellen in der Trägerflüssigkeit.

Als Ventile bzw. Mikroventile in den Einlass- und Auslasskanälen kommen dabei folgende Bauformen in Frage: elektromagnetisch, piezoelektrisch oder elektrostatisch betätigte Mikroventile, Mikrokugelventile, druckbetätigte Ventile.

Der Grundköper der erfindungsgemäßen Vorrichtung kann vieleckig, bevorzugt rechteckig sein. Der Grundkörper ist bevorzugt so geformt, dass die Verwendung mit einem Roboterarm einfach möglich ist, d.h. dass der Grundkörper eine leicht zu greifende Form besitzt. Der Grundkörper kann auch so gestaltet sein, dass er leicht mit der Aufnahme am Roboterarm fest verbunden werden kann, was beispielsweise durch Verschraubung erfolgen kann. Weiterhin kann das Zellsammlermodul mittig angeordnet sein.

Die Vorrichtung weist mindestens einen Einlasskanal für einen Mantelstrom auf, der in den mindestens einen Einlasskanal für die Trägerflüssigkeit mündet. Durch den Mantelstrom kann eine Vereinzelung der Zellen, die durch den Einlasskanal für die Trägerflüssigkeit hinzugefügt werden, ermöglicht werden.

Insbesondere kann das Zellsammlermodul aus einem Zellsammler und einem Dosierventilteilgehäuse aufgebaut sein. Diese können beispielsweise einzeln oder als Modul ausgetauscht werden.

Bevorzugt ist das Dosierventil aus einem Gegenstück und einer Membran aufgebaut. Somit wir ein unerwünschtes Tropfen der Vorrichtung vermieden. Weiterhin ist ein derartiges Dosierventil einfach zu Reinigen.

Der Zellsammler der erfindungsgemäßen Vorrichtung weist bevorzugt eine Membran, die gegebenenfalls elastisch ist, und das Dosierventilteilgehäuse ein Gegenstück auf, das insbesondere kegelförmig, kugelförmig oder zylinderförmig sein kann, wobei die Membran bevorzugt mit einer Vorspannung am Gegenstück anliegt.

Dabei können das Dosierventilteilgehäuse, die Membran und/oder das Gegenstück zumindest teilweise mit einer hydrophoben oder hydrophilen Beschichtung versehen sein. Weiterhin können das Dosierventilteilgehäuse, die Membran und/oder das Gegenstück auch aus einem hydrophoben oder hydrophilen Material gefertigt sein. Dadurch wird, in Abhängigkeit von den eingesetzten Medien, ein besseres Ablösen des Tropfens ermöglicht. Die Verwendung von hydrophoben oder hydrophilen Materialien ist abhängig von der Anwendung, d.h. z.B. davon, welche Art von Trägerflüssigkeit bzw. Dosiermedium verwendet wird.

Folglich kann, abhängig von den zu dosierenden Zellen (Suspensionskultur, adhärente Zellen) und den eingesetzten Medien, eine geeignete Beschichtung bzw. geeignetes Material für das Dosierventilteilgehäuse, die Membran bzw. das Gegenstück ausgewählt werden.

Bevorzugt weist das Dosierventilteilgehäuse auf seiner, dem Zellsammler zugewandten Seite, mittig einen Ventilkegel als Gegenstück auf sowie 1 bis 30 Bohrungen für das Dosiermedium, die außerhalb einer sichelartigen Nut zur Verankerung der Zellsammelstruktur angeordnet sind.

Der Ventilkegel verschließt hierbei das Dosierventil im Zusammenspiel mit der elastischen Membran, die unter Vorspannung am Ventilkegel anliegt, sofern Zellen gesammelt bzw. die Vorrichtung gereinigt wird. Die sichelartige Nut dient einer stabilen Verankerung der Zellsammelstruktur. Weiterhin dient die sichelartige Nut der Stabilisierung der Zellsammelstruktur und des Weiteren als Verdrehsicherung zwischen Dosierventilteilgehäuse und Zellsammler.

Weiterhin können das Dosierventilteilgehäuse und/oder der Zellsammler modular austauschbar sein. Dadurch kann zum einen beispielsweise nach einer Kontamination, ein schneller Austausch des Zellsammler bzw. des Dosierventils erfolgen, zum anderen ist die Vorrichtung dadurch flexibel für verschiedenste Zelltypen nutzbar, da je nach Zellgröße bzw. Zelltyp andere Zellsammler benötigt werden. Dazu können Zellsammler und Dosierventilteilgehäuse mit unterschiedlicher Geometrie in den Grundkörper eingesetzt werden.

Des Weiteren kann die Geometrie des Zellsammlers die maximale Anzahl der zu dosierenden Zellen über den Radius der Filter- bzw. Zellsammelstruktur bestimmen. In diesem Zusammenhang ist auch auf die Möglichkeit hinzuweisen, dass der Raum zwischen der Zellsammelstruktur und dem Gegenstück zur Membran des Dosierventils variiert werden kann und somit eine maximal zu dosierende Zellzahl beeinflusst. Da verschiedene Zelltypen verschiedene Größen aufweisen, ist es erforderlich, die Zellsammelstrukturen der jeweiligen Zellsammler an die Zellen anzupassen, d.h. die Abstände zwischen den kammartigen Erhöhungen der Zellsammelstruktur müssen zum jeweils zu dosierenden Zelltyp passen.

Bevorzugt ist das Zellsammlermodul zumindest teilweise mit mindestens einem Material beschichtet, insbesondere nicht-adhäsiven Beschichtungen, enthaltend oder bestehend aus Polyethylenglycol (PEG), Poly(2-hydroxy-ethyl-methyl(meth)acrylat) (Poly(HEMA)), Silikon, Polymethyl(meth)acylat (PMMA), Polyacrylamid, biokombatiblen Beschichtungen, insbesondere Laminin, Fibronectin, CollagenI/III/IV, Lysin bzw. behandeltem Polystyrol. Gegebenenfalls kann eine Haftvermittlungsschicht notwendig sein, die beispielsweise zwischen dem Zellsammler und der nicht-adhäsiven Beschichtung angeordnet ist. Weiterhin kann zusätzlich zur Beschichtung oder anstelle einer Beschichtung eine Plasmabehandlung des Zellsammlers zumindest teilweise erfolgen.

Durch diese Beschichtungen wird beispielsweise verhindert, dass adhärent wachsende Zellen im Zellsammler verbleiben. Folglich dient die Beschichtung oder die Plasmabehandlung der Oberflächenoptimierung und damit der Verringerung einer Fehlerwahrscheinlichkeit in Bezug auf die Zellzahl im dosierten Tropfen.

Der Grundkörper kann eine Sicherung zur Verhinderung eines Verdrehens des Zellsammlermoduls im Grundkörper aufweisen, durch die ein Verdrehen des Zellsammlermoduls gegenüber dem Grundkörper unterbunden werden kann. Diese verbessert insbesondere den Sitz des Zellsammlers sowie des Dosierventilteilgehäuses, also des Zellsammlermoduls, im Grundkörper und ermöglicht darüber hinaus eine optimale Dichtigkeit des Systems. Insbesondere kann die Sicherung zur Verhinderung eines Verdrehens so ausgebildet sein, dass eine Montage des Zellsammlers bzw. des Zellsammlermoduls in falscher Position unmöglich ist.

Erfindungsgemäß ist ein Verfahren zur Dosierung von Zellen, wobei die Trägerflüssigkeit mit den zu dosierenden Zellen durch den Einlasskanal für die Trägerflüssigkeit in Richtung Zellsammler fließt, wobei mittels der Zellerkennungsvorrichtung die Zellzahl ermittelt wird, sich die Zellen im Zellsammlermodul ansammeln und zumindest ein Teil der Trägerflüssigkeit durch den Auslasskanal für die Trägerflüssigkeit abfließt, während das Ventil im Auslasskanal für die Trägerflüssigkeit geöffnet ist und das Ventil im Auslasskanal für die Reinigung geschlossen ist und, nach Erreichen der zu dosierenden Zellzahl, die Steuerung bewirkt, dass ein Impuls auf den Einlasskanal des Dosiermediums gegeben wird und davor oder gleichzeitig das Ventil im Auslasskanal für die Trägerflüssigkeit sowie das Ventil im Einlasskanal für die Trägerflüssigkeit geschlossen wird oder die Diffusorstruktur als passives Ventil wirkt, wobei das Ventil im Auslasskanal für die Reinigungsflüssigkeit geschlossen bleibt, durch den Einlasskanal für das Dosiermedium das Dosiermedium hinzugefügt wird und mindestens eine Zelle in einem Tropfen abgegeben wird.

Weiterhin kann die Druckwelle bzw. der Impuls im Dosiermedium beispielsweise piezoelektrisch oder durch ein Elektroventil bzw. ein Magnetventil erzeugt werden. Auch eine mechanische Erzeugung der Druckwelle bzw. des Impulses wäre denkbar.

Hierbei werden zuerst die Zellen gezählt und im Zellsammler angehäuft. Wenn die gewünschte Zellzahl erreicht ist, werden die Zellen in einem Tropfen dosiert. Die zusätzliche Reinigungsfunktion ermöglicht, dass die Struktur des Zellsammlers von Zellrückständen und anderen Verunreinigungen befreit werden kann, oder, falls mehr Zellen als gewünscht im Zellsammler angehäuft wurden, diese aus dem Zellsammler herauszuspülen.

Weiterhin kann durch den Einlasskanal für den Mantelstrom ein weiteres Medium zugegeben werden, das einer Vereinzelung der Zellen dient, und eine Anordnung der Zellen hintereinander im Einlasskanal für die Trägerflüssigkeit bewirkt.

Dadurch wird eine verbesserte Zählung der Zellen durch die anschließend zu passierende Zellerkennungsvorrichtung ermöglicht. Die Zellerkennungsvorrichtung kann die durchströmenden Zellen z.B. über eine optische Messung, Impedanzmessung oder Kapazitätsmessung erkennen und das Ergebnis an die Steuerung weitergeben. Weiterhin sind die Strecke von der Zellerkennungsvorrichtung zum Zellsammler sowie die Fließgeschwindigkeit bekannt, so dass durch die Steuerung berechnet werden kann, wie viele Zellen sich zu welchem Zeitpunkt im Zellsammler befinden. Dieser Vorgang wird als Zellsammlung bezeichnet.

Ist die gewünscht Zellzahl im Zellsammler erreicht, so löst die Steuerung den Dosiervorgang aus. Dazu wird der Auslasskanal für die Trägerflüssigkeit mit einem Ventil verschlossen und ein Impuls auf den Einlasskanal für das Dosiermedium gegeben, wodurch die im Zellsammler vorhandenen Zellen durch das Dosierventil abgegeben werden. Dabei ist das Volumen der Dosierflüssigkeit regelbar, so dass sich ein Tropfen mit kontrolliertem Volumen und kontrollierter Zellzahl ergibt. Die Diffusorstruktur bzw. das Ventil im Einlasskanal für die Trägerflüssigkeit wirkt dabei ähnlich wie ein Rückschlagventil und verhindert, dass Zellen aus der Zellsammelstruktur zurück in den Einlasskanal für die Trägerflüssigkeit gelangen können bzw. sich die Druckwelle zu stark in den Einlasskanal für die Trägerflüssigkeit fortsetzt. Üblicherweise werden die Zellen in einer Mischung aus Trägerflüssigkeit, Dosiermedium und gegebenenfalls Flüssigkeit aus dem Mantelstrom abgegeben.

Es ist jedoch auch möglich, dass das Trägermedium bzw. die Flüssigkeit aus dem Mantelstrom, wenn erforderlich, vor einer Zudosierung des Dosiermediums, beispielsweise durch den Auslasskanal für die Trägerflüssigkeit, entfernt werden oder abfließen.

Nach dem Dosieren wird der Auslasskanal für die Trägerflüssigkeit wieder freigegeben und der Zellsammler kann erneut mit der gewünschten Zellzahl befüllt werden.

Es kann im Anschluss und/oder während des Dosiervorgangs, sofern die Zellzahl überschritten wurde, ein Reinigungsschritt durchgeführt werden, wobei durch den Einlasskanal für das Dosiermedium Reinigungsflüssigkeit eingeleitet wird, und das Ventil des Auslasskanals für die Trägerflüssigkeit sowie das Dosierventil geschlossen sind und die Flüssigkeit durch den Auslasskanal für die Reinigungsflüssigkeit abfließt. Die Reinigungsflüssigkeit kann auch identisch mit dem Dosiermedium oder der Trägerflüssigkeit sein.

Ein Reinigungsschritt kann beispielsweise dann erforderlich werden, wenn der Zellsammler bzw. das Zellsammlermodul von Verunreinigungen wie Zellrückständen oder Proteinen zu befreien ist, sowie um Zellen aus dem Zellsammler zu entfernen, wenn zu viele Zellen dort angesammelt wurden. Zum Reinigen wird der Auslasskanal für die Trägerflüssigkeit verschlossen und der Reinigungskanal geöffnet. Dann wird durch den Einlasskanal für das Dosiermedium eine Flüssigkeit, insbesondere eine Reinigungsflüssigkeit, geleitet, die beispielsweise Rückstände aus der Zellsammelstruktur durch den Reinigungskanal abfließen lässt.

Für das erfindungsgemäße Verfahren können als Trägerflüssigkeit Zellkulturmedium, Salzlösungen, gepufferte Lösungen, isotonische Kochsalzlösung, 2(4-(2-Hydroxyethyl)-1-piperazinyl)-ethansulfonsäure (HEPES)-gepufferte Lösungen, Tris(hydroxy-methyl)-aminomethan (TRIS)-gepufferte Lösungen, Natriumcitrat enthaltenden Lösungen, Trypsin enthaltende Lösungen, Ethylendiamintetraessigsäure (EDTA) enthaltende Lösungen, Antibiotika enthaltende Lösungen, Antimycotika enthaltende Lösungen oder Mischungen hiervon eingesetzt werden.

Als Dosiermedium werden bevorzugt Zellkulturmedium, Salzlösungen, isotonische Kochsalzlösung, 2(4-(2-Hydroxyethyl)-1-pipera-zinyl)-ethansulfonsäure (HE-PES)-gepufferte Lösungen, Tris(hydroxymethyl)-aminomethan (TRIS)-gepufferte Lösungen, Fluoreszenzmarker enthaltende Lösungen, 3-(4,5-Dimethylthiazol-2-yl)-2,5-diphenyltetrazoliumbromid (MTT) enthaltende Lösungen oder Mischungen hiervon eingesetzt.

Gepufferte Salzlösungen (balanced salt solutions) werden benutzt als
- In Verbindung mit Kohlenhydraten (Glucose) wird die hauptsächliche Energiequelle für den Metabolismus der Zellen geliefert;
- Transport- oder Verdünnungsmedium zur Aufrechterhaltung der osmotischen Balance;
- Versorgung der Zellen mit Wasser und bestimmten anorganischen Ionen, essentiell für den normalen Zellmetabolismus;
- Puffersystem zur Erhaltung des physiologischen pH Bereichs.

### Gebräuchliche Salzlösungen sind:

- Dulbecco's Phosphate Buffered Saline
- Earle's Balanced Salts
- Hanks' Balanced Salts
- Tyrode's Salts
- Phosphate Buffered Saline
wie auch viele spezifischen Modifikationen dieser Lösungen (oft mit dem Zusatz "modified") und seltener verwendete gepufferte Salzlösungen (Alsever's Solution, Gey's Balanced Salt Solution, Kreb's Henseleit Buffer Modified, etc.)

### Klassische Zellkulturmedien sind:

### Gebräuchliche Medien

- Medium 199: klassisches Gewebe-Kultur Medium; lieferte eine der ersten vollständig definierten Nährstoffquellen für die Zellkultur;
- DMEM (Dulbecco's Modified Eagle's Medium): Modifizierung des Basal Medium Eagle (BME) zur Verwendung mit embryonalen Mauszellen; große Anzahl an weiteren Modifikationen für spezifische Anwendungen/spezielle Zelllinien;
- MEM (Minimum Essential Medium Eagle): Weit verbreitetes synthetisches Zellkulturmedium mit vielen Variationen/Modifikationen zum ursprünglichen Rezept zur Verwendung für verschiedene Applikationen/Zelltypen;
- RPMI-1640 (Roswell Park Memorial Institute): Ein Bicarbonat-Puffersystem, angereichert mit Vitaminen und Aminosäuren, häufig benutzt zur Kultur von primären Zellen, hauptsächlich Leukozyten; heutzutage allerdings zur Anzucht von vielen Ze-Illinien verwendet.
sowie viele weitere spezielle bzw. seltener verwendete Medien wie: Ames' Media, BGJb Medium (Fitton-Jackson Modification), Click's Medium, CMRL-1066 Medium, Fischer's Medium, Glascow Minimum Essential Medium (GMEM), Iscove's Modified Dulbecco's Medium (IMDM), L-15 Medium (Leibovitz), McCoy's 5A Modified Medium, NCTC Medium, Swim's S-77 Medium, Waymouth Medium und William's Medium E.

### Spezialmedien

Weiterentwickelte Medien mit niedrigerem Serum- oder Glutamin-Verbrauch oder für spezielle Applikationen abgewandelte Rezepturen. In diesem Zusammenhang ist das folgende Zellkulturmedium nur beispielhaft zu sehen, da es auch hier viele weitere Zusammensetzungen und Medien gibt.
- MCDB Medium (Ham et. al.): low-protein- und lowserum-Medium mit spezifischer Rezeptur auf einzelne Zelllinien abgestimmt (embryonale Kulturen, etc.)

### Serum-freie Medien

- Chemisch-definierte Medien und Zusätze, die ohne die Zugabe von tierischen Zusätzen, wie Serum (FCS etc.), eingesetzt werden können. Inhaltsstoffe sind oft auf bestimmte Zelllinien abgestimmt und besitzen ein eingeschränktes Anwendungsgebiet.

Bekannte Zelllinien, für die Serum-freie Medien entwickelt wurden, sind: CHO Zellen (Chinese Hamster Ovary), Hybridoma-Systeme und viele mehr.

### Weitere Kultur-Medien für Mikroorganismen, Pflanzen und Pilze

Abhängig von Art und Gruppe der Mikroorganismen (Hefe, Bakterien, Pilze) gibt es eine unüberschaubare Anzahl von Medientypen und -formulierungen.

Die bekannteste Formulierung für ein Medium zur Bakterienanzucht ist dabei LB-Medium.

Für das erfindungsgemäße Verfahren können als Trägerflüssigkeit und als Dosiermedium identische Lösungen eingesetzt werden. Weiterhin kann als Reinigungsflüssigkeit eine mit der Trägerflüssigkeit und/oder dem Dosiermedium identische Lösung eingesetzt werden. Die Auswahl der genannten Lösungen, Medien etc. ist dabei beispielhaft zu sehen, da es darüber hinaus noch eine Vielzahl weiterer einsetzbarer Lösungen und Medien gibt.

Es ist jedoch auch möglich, dass als Trägerflüssigkeit und als Dosiermedium verschiedene Lösungen eingesetzt werden. Dies ist insbesondere dann von Bedeutung, wenn beispielsweise zellschädigende Zusätze eingesetzt werden müssen. Werden diese dem Dosiermedium beigefügt, werden die Zellen folglich ein deutlich kürzeres Zeitintervall mit diesen toxischen Zusatzstoffen in Kontakt gebracht im Vergleich zu dem Fall, in dem diese Zusatzstoffe bereits in der Trägerflüssigkeit enthalten sind.

Weiterhin kann die Zudosierung bestimmter Substanzen auch dann von Interesse sein, wenn ab dem Beginn der Zudosierung des Dosiermediums und einem bestimmten Substrat eine Kinetik, beispielsweise bezüglich einer Verstoffwechselung des Substrats, aufgenommen werden soll.

Erfindungsgemäß wird die Vorrichtung zur Dosierung von menschlichen Zellen, tierischen Zellen, Prokaryonten, Eukaryonten und/oder pflanzlichen Zellen verwendet.

Hier sind beispielhaft zu nennen:
- Primäre Zelllinien:
   Alle Zellen, die direkt aus dem menschlichen oder tierischen Körper entfernt wurden. Sie können oftmals nur wenige Passagen kultiviert werden.
   Dazu gehören: Lymphoide Zellen (weiße Blutzellen), Hepatozyten (Leberzellen), etc.
   Sie können grob in adhärente Linien (gewebeformend) und Suspensions-Zellen (oftmals Blutzellen) eingeteilt werden, wobei mehr als 90 % aller primären Zelllinien adhärent sind.
- Immortalisierte Zelllinien:
   Diese Zelllinien wachsen und vermehren sich unendlich in vitro, so lange die richtigen Kulturbedingungen eingehalten werden. Dazu gehören auch, aber nicht ausschließlich, kanzerogene Zellen und Tumorzellen.
   Dazu gehören: HeLa (adhärent, nicht kanzerogen), MCF-7 (adhärent, kanzerogen), JURKAT (Suspension, kanzerogen), etc.
- Werden andere Modelorganismen (Mus musculus, Danio rerio) verwendet, wird eine analoge Einteilung vorgenommen. Unizelluläre Organismen sind davon ausgenommen.
- Niedrige Eukaryoten (z.B. Pilze (Fungi), Saccharomyces cerevisae, etc.), Prokaryoten (Escherichia coli, Staphylococcus aureus, etc.) aber auch viele Pflanzenzellen können analog verwendet werden.

Anhand der nachfolgenden Figuren 1 bis 11 soll der anmeldungsgemäße Gegenstand näher erläutert werden, ohne diesen auf diese Varianten einzuschränken.

Es zeigt
- Figur 1: eine isometrische Ansicht der Oberseite der erfindungsgemäßen Vorrichtung;
- Figur 2: eine isometrische Ansicht der Unterseite der Zelldosierungsvorrichtung;
- Figur 3A: die erfindungsgemäße Vorrichtung in einer Ansicht von unten;
- Figur 3B: die Vorrichtung in einer Seitenansicht von rechts;
- Figur 3C: die Vorrichtung in der Vorderansicht;
- Figur 3D: die Vorrichtung in der Seitenansicht von links;
- Figur 3E: die Vorrichtung in der Rückansicht;
- Figur 3F: die Vorrichtung in einer Ansicht von oben;
- Figur 4A: eine Vorderansicht der Vorrichtung;
- Figur 4B: einen horizontalen Schnitt durch die Vorrichtung während des Zellsammelvorgangs;
- Figur 5A: die Vorderansicht der Vorrichtung;
- Figur 5B: den horizontalen Schnitt durch die Vorrichtung während des Dosiervorgangs;
- Figur 5C: einen vergrößerten Ausschnitt aus Figur 5B;
- Figur 6A: die Vorderansicht der erfindungsgemäßen Vorrichtung;
- Figur 6B: einen horizontalen Schnitt durch die erfindungsgemäße Vorrichtung während des Reinigungsvorgangs;
- Figur 7A: eine Vorderansicht der erfindungsgemäßen Vorrichtung;
- Figur 7B: einen vertikalen Schnitt durch die erfindungsgemäße Vorrichtung;
- Figur 8: den Zellsammler in verschiedenen Ansichten;
- Figur 8A: den Zellsammler in einer Ansicht von schräg oben;
- Figur 8B: den Zellsammler in einer Ansicht von schräg unten;
- Figur 8C: den Zellsammler in einer Ansicht von oben;
- Figur 8D: einen horizontalen Schnitt durch Figur 8C;
- Figur 8E: einen vergrößerten Ausschnitt aus Figur 8D;
- Figur 9: das Dosierventilteilgehäuse in unterschiedlichen Ansichten;
- Figur 9A: das Dosierventilteilgehäuse in einer Ansicht von schräg oben;
- Figur 9B: das Dosierventilteilgehäuse in einer Ansicht von schräg unten;
- Figur 9C: das Dosierventilteilgehäuse in einer Ansicht von unten;
- Figur 9D: eine Seitenansicht von Figur 9c;
- Figur 9E: eine Ansicht von oben auf das Dosierventilteilgehäuse;
- Figur 10: eine Ansicht des Grundkörpers von schräg unten ohne Zellsammler und Dosierventilteilgehäuse;
- Figur 11: verschiedene Ansichten der Kombination aus Zellsammler und Dosierventilteilgehäuse als Zellsammlermodul;
- Figur: 11A eine Ansicht von oben auf ein Dosierventilteilgehäuse, das mit einem Zellsammler verbunden ist;
- Figur 11B: einen vertikalen Schnitt durch Figur 11A; und
- Figur 11C: zeigt einen horizontalen Schnitt durch Figur 11A.

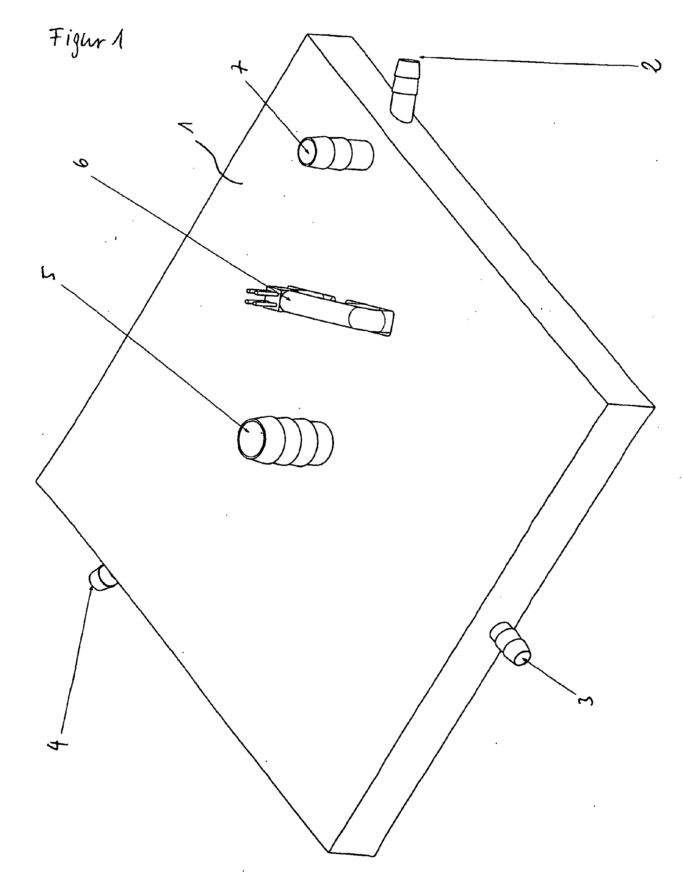

In Figur 1 ist eine Ansicht von schräg oben auf die erfindungsgemäße Vorrichtung dargestellt. Der Grundkörper 1 weist auf seiner Oberseite einen Einlasskanal für das Dosiermedium 5 sowie eine Zellerkennungsvorrichtung 6 auf. Der Einlasskanal für den Mantelstrom 7 ist senkrecht zum Einlasskanal für die Trägerflüssigkeit 2 angeordnet. An der gegenüber liegenden Seite des Einlasskanals für die Trägerflüssigkeit 2 ist der Auslass des Reinigungskanals 4 angeordnet. Der Auslasskanal für die Trägerflüssigkeit 3 ist an der angrenzenden Fläche angeordnet.

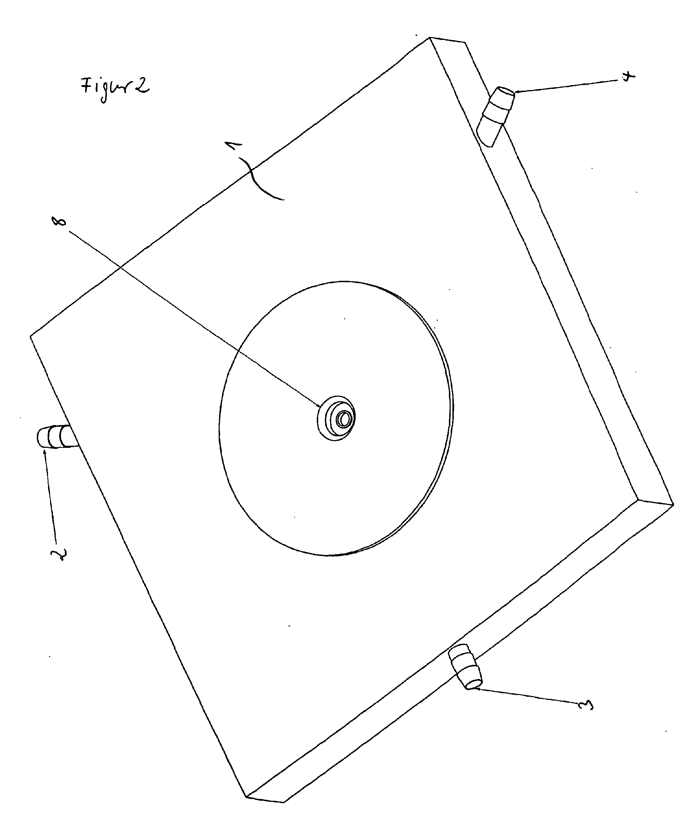

Figur 2 zeigt eine Ansicht von schräg unten. Der Auslass für die dosierten Zellen 8 ist in der Mitte des Grundkörpers 1 angeordnet. Der Einlasskanal für die Trägerflüssigkeit 2 liegt dem Auslass des Reinigungskanals 4 gegenüber. An der dazwischen liegenden Seite ist der Auslasskanal für die Trägerflüssigkeit 3 angeordnet.

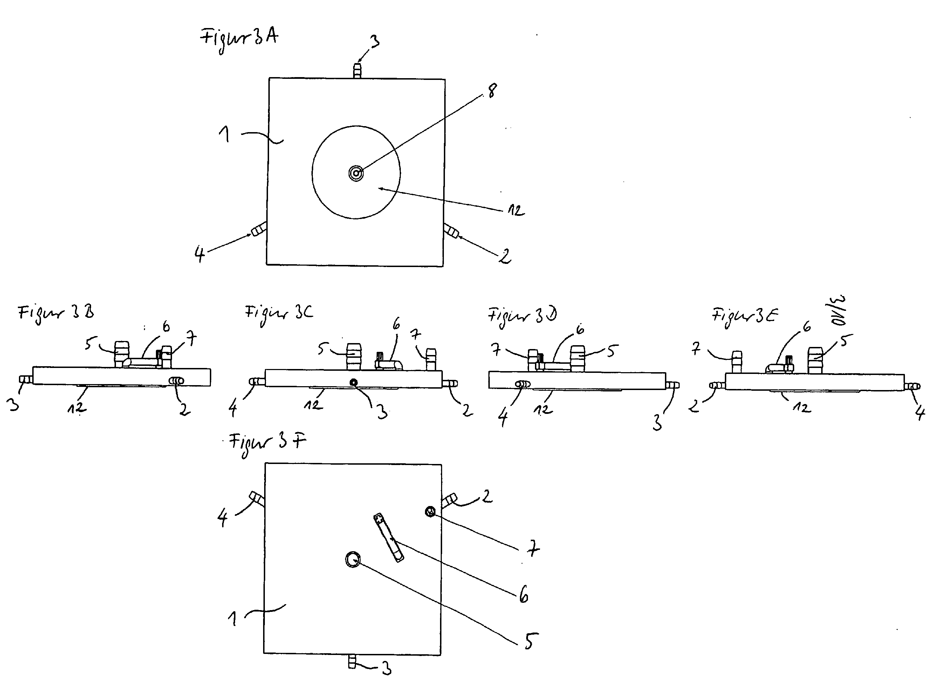

Figur 3A zeigt eine Unteransicht der erfindungsgemä-βen Vorrichtung zur Dosierung von Zellen. Der Einlasskanal für die Trägerflüssigkeit 2, der Auslasskanal für die Trägerflüssigkeit 3 sowie der Reinigungskanal 4 münden mittig in den Zellsammler 13, der in dieser Figur nicht dargestellt ist. Der Auslass für die dosierten Zellen 8 ist mittig in der Halteplatte des Zellsammlermoduls 12 im Grundkörper 1 angeordnet. Das Zellsammlermodul 23 besteht aus dem Zellsammler 13 und dem Dosierventilgehäuse 14.

Figur 3B zeigt die Seitenansicht von rechts. Auf der Oberseite des Grundkörpers 1 sind der Einlasskanal für das Dosiermedium 5, die Zellerkennungsvorrichtung 6 sowie der Einlasskanal für den Mantelstrom 7 angeordnet. Auf der Unterseite des Grundkörpers 1 ist die Halteplatte für das Zellsammlermodul 12 angeordnet. Der Auslasskanal für die Trägerflüssigkeit 3 weist nach links. Der Einlasskanal für die Trägerflüssigkeit 2 weist in dieser Ansicht nach vorne.

In Figur 3C ist eine Vorderansicht der erfindungsgemäßen Vorrichtung zur Dosierung von Zellen dargestellt. Auf der Oberseite des Grundkörpers 1 sind der Einlasskanal für das Dosiermedium 5, die Zellerkennungsvorrichtung 6 sowie der Einlass für den Mantelstrom 7 angeordnet. Der Reinigungskanalauslass 4 weist nach links, der Auslasskanal für die Trägerflüssigkeit 3 nach vorne und der Einlasskanal für die Trägerflüssigkeit 2 nach rechts. Unterhalb des Grundkörpers 1 ist die Halteplatte für das Zellsammlermodul 12 angeordnet.

Figur 3D zeigt eine Seitenansicht von links. Der Einlasskanal für den Mantelstrom 7, die Zellerkennungsvorrichtung 6 sowie der Einlasskanal für das Dosiermedium 5 sind auf der Oberseite des Grundkörpers 1 angeordnet. Der Auslass des Reinigungskanals 4 weist nach vorne und der Auslasskanal für die Trägerflüssigkeit 3 nach rechts. Die Halteplatte für das Zellsammlermodul 12 ist an der Unterseite des Grundkörpers 1 angeordnet.

Figur 3E zeigt eine Rückansicht der erfindungsgemäßen Vorrichtung. Der Einlasskanal für den Mantelstrom 7, die Zellerkennungsvorrichtung 6 sowie der Einlasskanal für das Dosiermedium 5 sind auf der Oberseite des Grundkörpers 1 angeordnet. Der Einlasskanal für die Trägerflüssigkeit 2 weist nach links und der Reinigungskanal 4 nach rechts. Auf der Unterseite des Grundkörpers 1 ist die Halteplatte für das Zellsammlermodul 12 angeordnet.

In Figur 3F ist eine Ansicht von oben auf die Vorrichtung dargestellt. Der Einlasskanal für das Dosiermedium 5 ist mittig auf dem Grundkörper 1 angeordnet. In ihn münden der Einlasskanal für die Trägerflüssigkeit 2, der Auslasskanal für die Trägerflüssigkeit 3 sowie der Reinigungskanal 4. Senkrecht zum Einlasskanal für die Trägerflüssigkeit 2 ist der Einlasskanal für den Mantelstrom 7 angeordnet. Weiterhin weist die erfindungsgemäße Vorrichtung auf der Oberseite des Grundkörpers eine Zellerkennungsvorrichtung 6 auf.

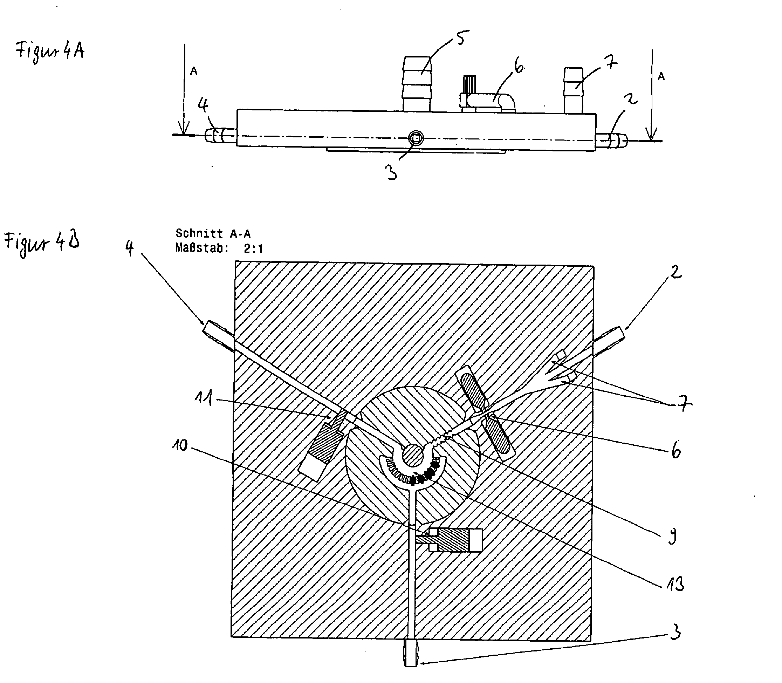

In Figur 4A ist eine Seitenansicht der erfindungsgemäßen Vorrichtung dargestellt. Der Einlasskanal für das Dosiermedium 5, die Zellerkennungsvorrichtung 6 sowie der Einlasskanal für den Mantelstrom 7 sind auf der Oberseite des Grundkörpers 1 angeordnet. Der Reinigungskanal 4 weist nach links, der Auslasskanal für die Trägerflüssigkeit 3 nach vorne und der Einlasskanal für die Trägerflüssigkeit 2 nach rechts.

In Figur 4B ist ein horizontaler Schnitt durch Figur 4A dargestellt. Dies zeigt die Ventilstellungen während des Zellsammelvorgangs. Durch den Einlasskanal für die Trägerflüssigkeit 2 werden die Zellen in den Zellsammler 13 gespült. Dabei ist das Ventil im Auslasskanal für die Reinigungsflüssigkeit 11 geschlossen und das Ventil im Auslasskanal für die Trägerflüssigkeit 10 geöffnet. Die Zellerkennungsvorrichtung 6 ist zwischen dem Einlass für die Trägerflüssigkeit 2 und dem Zellsammler 13 angeordnet. Weiterhin sind Einlasskanäle für den Mantelstrom 7, die in den Einlasskanal für die Trägerflüssigkeit 2 münden, dargestellt.

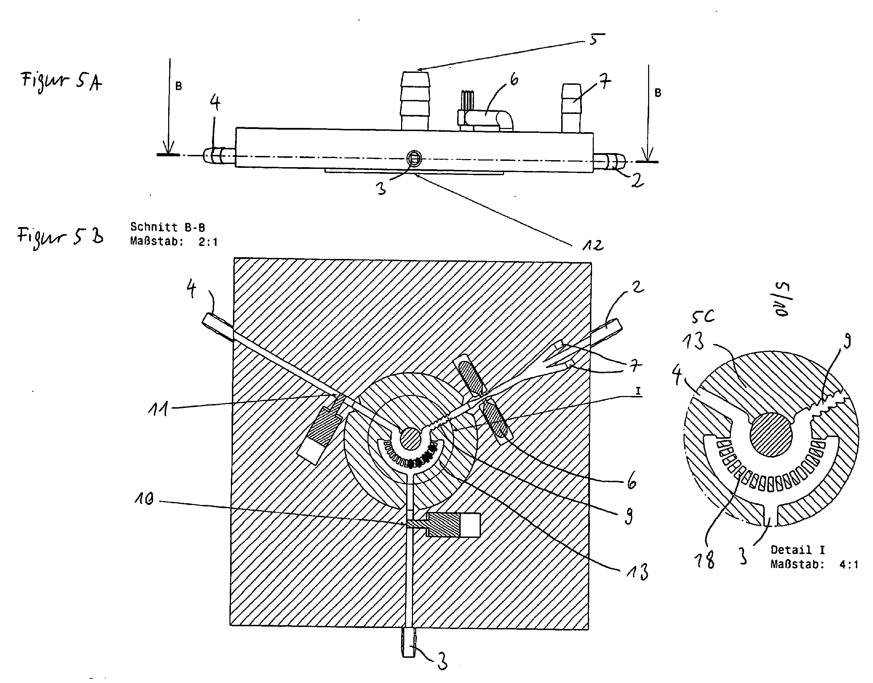

Figur 5A zeigt eine Seitenansicht der erfindungsgemä-βen Vorrichtung. Der Einlasskanal für das Dosiermedium 5, die Zellerkennungsvorrichtung 6 sowie der Einlasskanal für den Mantelstrom 7 sind auf der Oberseite des Grundkörpers 1 angeordnet. Der Auslass des Reinigungskanals 4 weist nach links, der Auslasskanal für die Trägerflüssigkeit 3 nach vorne und der Einlasskanal für die Trägerflüssigkeit 2 nach rechts.

Figur 5B zeigt einen horizontalen Schnitt durch Figur 5A, während des Dosiervorgangs. Hierbei sind sowohl das Ventil im Auslasskanal für die Reinigung 11 sowie das Ventil im Auslasskanal für die Trägerflüssigkeit 10 geschlossen. Die Diffusorstruktur 9 ist ein integraler Bestandteil des Zellsammlers 13, die benachbart zur Zellsammelstruktur 18 angeordnet ist. Eine Zellzählung erfolgt über die Zellerkennungsvorrichtung 6, die an dem Einlasskanal für die Trägerflüssigkeit 2 angeordnet ist. Durch den Einlasskanal für den Mantelstrom 7 kann Medium zur Vereinzelung der Zellen hinzugefügt werden.

In Figur 5C ist ein Ausschnitt des Zellsammlermoduls 23 vergrößert dargestellt. Dieser weist eine Zellsammelstruktur 18 auf. Nach unten zeigend ist der Auslasskanal für die Trägerflüssigkeit 3 dargestellt. Der Einlasskanal für die Trägerflüssigkeit 2, der über die Diffusorstruktur 9 in den Zellsammler 13 mündet, weist nach rechts. Der Auslass für den Reinigungskanal 4 weist nach links.

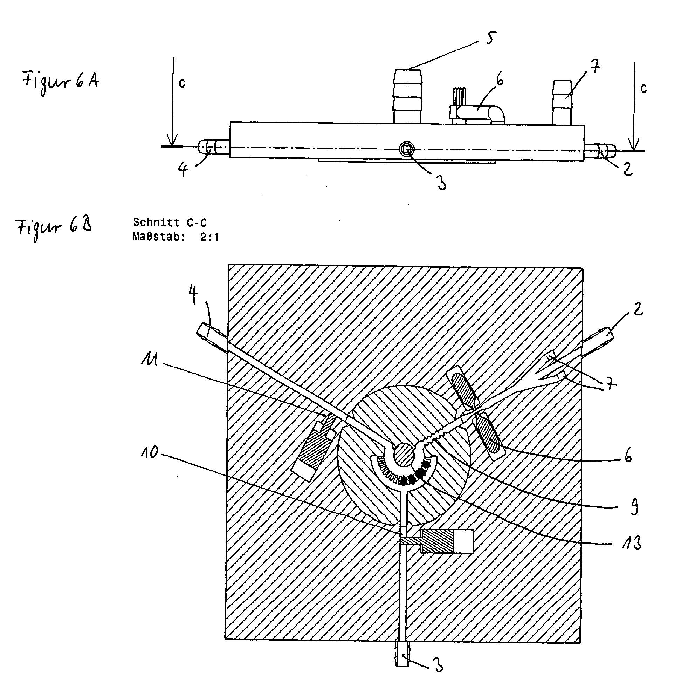

In Figur 6A ist eine Seitenansicht der erfindungsgemäßen Vorrichtung dargestellt. Der Einlasskanal für das Dosiermedium 5, die Zellerkennungsvorrichtung 6 sowie der Einlasskanal für den Mantelstrom 7 sind auf der oberen Seite des Grundkörpers 1 angeordnet. Der Reinigungskanalauslass 4 weist nach links, der Auslasskanal für die Trägerflüssigkeit 3 nach vorne und der Einlasskanal für die Trägerflüssigkeit 2 nach rechts.

In Figur 6B ist der horizontale Schnitt durch Figur 6A während des Reinigungsvorgangs dargestellt. Dabei ist das Ventil im Auslasskanal für die Trägerflüssigkeit 10 geschlossen und das Ventil im Auslasskanal für die Reinigung 11 geöffnet. Während der Reinigung wird das Reinigungsmedium durch den Einlasskanal für das Dosiermedium 5 in den Zellsammler 13 gespült und, nachdem es den Zellsammler 13 passiert hat, über den Reinigungskanal 4 abgeführt.

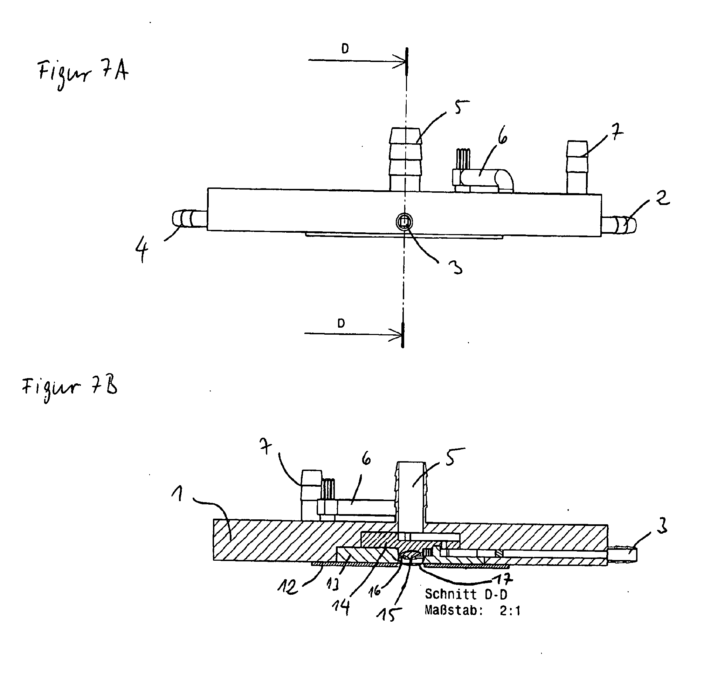

Figur 7A zeigt eine Seitenansicht der erfindungsgemä-βen Dosiervorrichtung. Der Einlasskanal für das Dosiermedium 5, die Zellerkennungsvorrichtung 6 sowie der Einlasskanal für den Mantelstrom 7 sind auf der oberen Seite des Grundkörpers 1 angeordnet. Der Reinigungskanal 4 weist nach links, der Auslasskanal für die Trägerflüssigkeit 3 nach vorne und der Einlasskanal für die Trägerflüssigkeit 2 nach rechts.

Figur 7B zeigt den vertikalen Schnitt durch Figur 7A. Hierbei weist der Auslasskanal für die Trägerflüssigkeit 3 nach rechts. Unterhalb des Einlasskanals für das Dosiermedium 5 ist das Dosierventilteilgehäuse 14 angeordnet, das als Gegenstück 15 einen Ventilkegel aufweist. Die Membran 16 ist im Zellsammler 13 integriert. Die Membran 16 liegt am Ventilkegel 15 an. Sie wird üblicherweise nur im Falle des Dosiervorgangs durch die Druckwelle nach dem Impuls auf den Einlasskanal 5 vom Ventilkegel 15 abgehoben, so dass sich der Tropfen lösen kann. Der Zellsammler 13, der an das Dosierventilteilgehäuse 14 direkt angrenzt, wird über die Halteplatte des Zellsammlermoduls 12 an den Grundkörper 1 fixiert. Das Zellsammlermodul ist aus dem Zellsammler 13 und dem Dosierventilteilgehäuse 14 aufgebaut. Das Dosierventil 17 setzt sich aus dem Ventilkegel 15 und der Membran 16 zusammen.

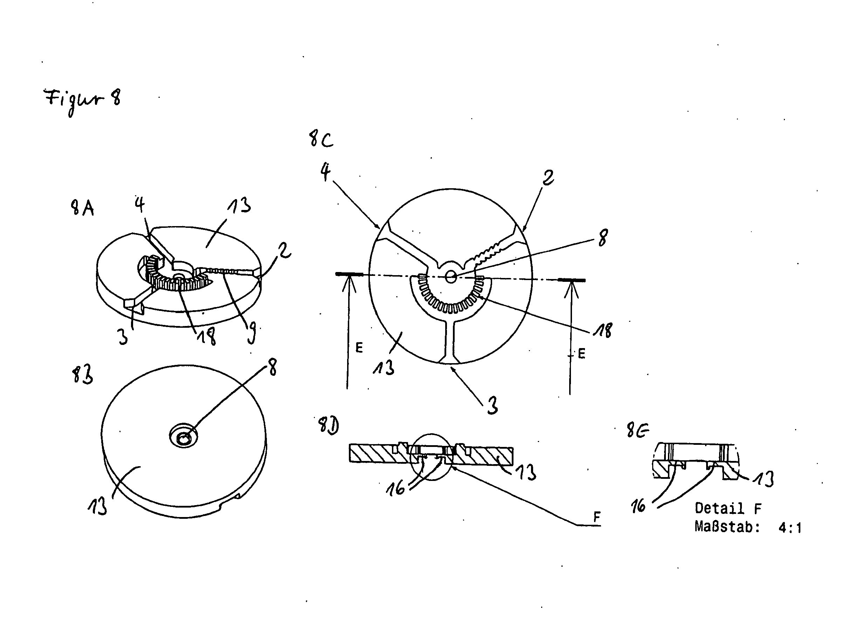

Figur 8 zeigt verschiedene Ansichten des Zellsammlers.

In Figur 8A ist eine Ansicht von schräg oben dargestellt. Hierbei weist der Einlasskanal für die Trägerflüssigkeit 2 nach rechts und der Auslasskanal für die Trägerflüssigkeit 3 nach links vorne. Der Auslass für den Reinigungskanal 4 weist nach links hinten. In der Mitte des Zellsammlers 13 ist die Zellsammelstruktur 18 dargestellt.

Figur 8B zeigt eine Ansicht von schräg unten. Der Auslass für die dosierten Zellen 8 ist in der Mitte des Zellsammlers 13 angeordnet.

Figur 8C zeigt eine Ansicht des Zellsammlers 13 von oben. In der Mitte des Zellsammlers 13 ist der Zellauslass 8 dargestellt. Weiterhin ist die Zellsammelstruktur 18 halbkreisförmig angeordnet. Der Einlasskanal für die Trägerflüssigkeit 2 zeigt nach rechts oben, der Auslasskanal für die Trägerflüssigkeit 3 nach unten und der Auslass des Reinigungskanals 4 nach links oben.

Figur 8D zeigt einen horizontalen Schnitt durch Figur 8C. Hierbei ist mittig des Zellsammlers 13 die Membran 16 dargestellt, die in den Zellsammler 13 integriert ist.

Figur 8E zeigt den markierten Ausschnitt F aus Figur 8D. Hier ist vergrößert die in den Zellsammler 13 integrierte Membran 16 dargestellt.

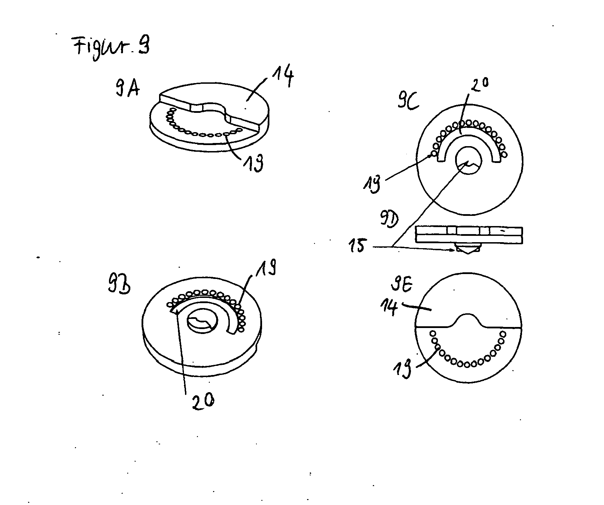

Figur 9 zeigt verschiedene Ansichten des Dosierventilteilgehäuses 14.

In Figur 9A ist eine Ansicht von schräg oben dargestellt. Das Dosierventilteilgehäuse 14 weist Bohrungen für das Dosiermedium 19 auf.

In Figur 9B ist das erfindungsgemäße Dosierventilteilgehäuse 14 in einer Ansicht von schräg unten dargestellt. Hier sind die Bohrungen für das Dosiermedium 19 erkennbar sowie die sichelförmige Nut 20, die einer Verankerung der Zellsammelstruktur 18 dient.

In Figur 9C ist eine Ansicht des Dosierventilteilgehäuses 14 von unten dargestellt. Die Bohrungen für das Dosiermedium 19 grenzen direkt an die sichelförmige Nut 20 zur Verankerung der Zellsammelstruktur 18 an. Mittig ist der Ventilkegel 15 dargestellt.

Figur 9D zeigt eine Seitenansicht von Figur 9C. Der Ventilkegel 15 befindet sich mittig auf der unteren Seite des Dosierventilteilgehäuses 14.

Figur 9E zeigt das Dosierventilteilgehäuse 14 in einer Ansicht von oben. Hier sind wiederum die Bohrungen für das Dosiermedium 19 erkennbar.

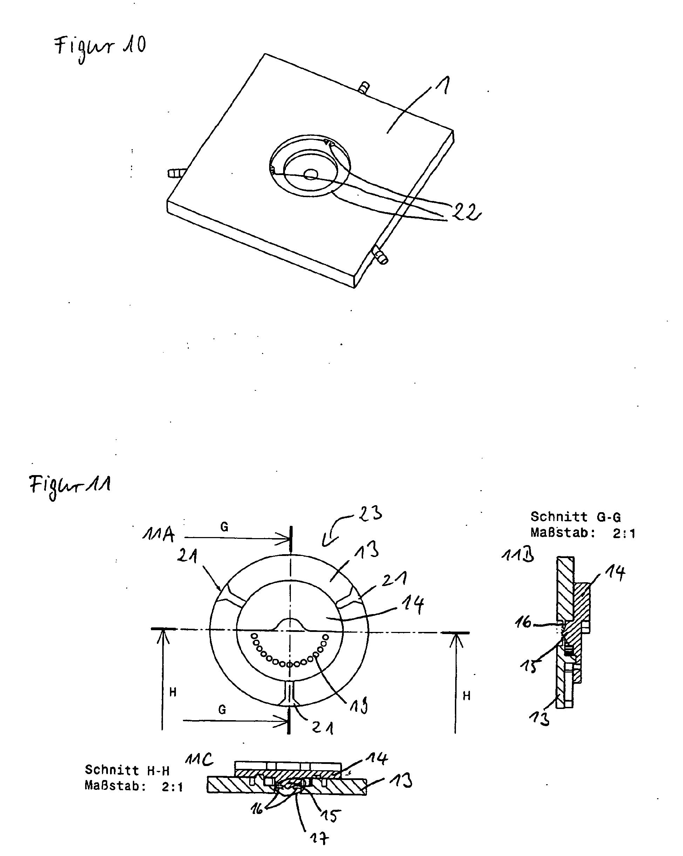

Figur 10 zeigt den Grundkörper 1 in einer Ansicht von schräg unten. Hier ist die Aussparung für das Zellsammlermodul 23 aus Zellsammler 13 und Dosierventil 14 dargestellt. Die Sicherung gegen ein Verdrehen 22 dient einer stabilen Fixierung des Zellsammlers 13 in dem Grundkörper 1.

Figur 11 zeigt das Dosierventilteilgehäuse 14 und den Zellsammler 13 in zusammengebauter Form als Zellsammlermodul 23.

In Figur 11A ist eine Ansicht von oben dargestellt. Der Zellsammler 14, der Bohrungen für das Dosiermedium 19 aufweist, ist auf dem Zellsammler 13 angeordnet. Der Zellsammler 13 weist drei Aussparungen 21 für die Sicherung gegen ein Verdrehen 22 auf. Auf diese Weise wird eine stabile Befestigung des Zellsammlers 13 sowie des Zellsammlermoduls 23 in dem Grundkörper 1 gewährleistet.

Figur 11B zeigt einen vertikalen Schnitt durch das in Figur 11A dargestellte Zellsammlermodul 23. Das rechts dargestellte Dosierventilteilgehäuse 14 weist einen Ventilkegel als Gegenstück 15 auf, der direkt an die Membran 16 angrenzt. Weiterhin ist der Zellsammler 13, der mit dem Dosierventilteilgehäuse 14 verbunden ist, dargestellt.

Figur 11C zeigt einen horizontalen Schnitt durch Figur 11A. Das Dosierventilteilgehäuse 14, das einen Ventilkegel 15 aufweist, ist oberhalb des Zellsammlers 13 angeordnet. Die Membran 16 ist in der Mitte des Zellsammlers 13 dargestellt. Das Dosierventil 17 besteht aus der im Zellsammler 13 integrierten Membran 16 und dem Ventilkegel als Gegenstück 15.

## Patentansprüche

1. Vorrichtung zur Dosierung von Zellen bestehend aus einem Grundkörper (1), wobei in mindestens einer Ebene des Grundkörpers (1) mindestens ein Einlass- (2) und Auslasskanal (3) für die Trägerflüssigkeit, sowie mindestens ein Reinigungskanal (4) und im Wesentlichen senkrecht zu den in dieser Ebene angeordneten Kanälen (2, 3, 4) mindestens ein weiterer Einlasskanal (5) für das Dosiermedium vorgesehen ist und diese Kanäle (2, 3, 4, 5) in einem Zellsammlermodul (23) münden,
wobei das Zellsammlermodul (23) einen Zellauslass (8) mit einem Dosierventil (17) aufweist und
wobei eine Steuerung für die Auslösung des Dosiervorgangs und eine Zellerkennungsvorrichtung (6) vorgesehen sind, wobei die Zellerkennungsvorrichtung (6) auf oder am Grundkörper (1) angeordnet ist oder im Grundkörper (1) integriert ist, **dadurch gekennzeichnet, dass** die Vorrichtung mindestens einen Einlasskanal für einen Mantelstrom (7) aufweist, der in den mindestens einen Einlasskanal (2) für die Trägerflüssigkeit mündet.

2. Vorrichtung nach Anspruch 1, **dadurch gekennzeichnet, dass** die Kanäle (2, 3, 4) mindestens jeweils ein Ventil (10, 11) und/oder eine Diffusorstruktur (9) aufweisen.

3. Vorrichtung nach Anspruch 1 oder 2, **dadurch gekennzeichnet, dass** der Grundkörper (1) vieleckig, bevorzugt rechteckig, und das Zellsammlermodul (23) mittig angeordnet ist.

4. Vorrichtung gemäß einem der vorhergehenden Ansprüche, **dadurch gekennzeichnet, dass** die Vorrichtung mindestens einen Einlasskanal (7) für einen Mantelstrom aufweist, der in den mindestens einen Einlasskanal (2) für die Trägerflüssigkeit mündet.

5. Vorrichtung gemäß einem der vorhergehenden Ansprüche, **dadurch gekennzeichnet, dass** das Zellsammlermodul (23) aus einem Zellsammler (13) und einem Dosierventilteilgehäuse (14) aufgebaut ist.

6. Vorrichtung gemäß einem der vorhergehenden Ansprüche, **dadurch gekennzeichnet, dass** das Dosierventil (17) aus einem Gegenstück (15) und einer Membran (16) aufgebaut ist.

7. Vorrichtung gemäß dem vorhergehenden Anspruch, **dadurch gekennzeichnet, dass** der Zellsammler (13) eine Membran (16), die gegebenenfalls elastisch ist, und das Dosierventilteilgehäuse (14) ein Gegenstück (15), das insbesondere kegelförmig, kugelförmig oder zylinderförmig ist, aufweist, wobei die Membran (16) bevorzugt mit einer Vorspannung am Gegenstück (15) anliegt.

8. Vorrichtung gemäß einem der beiden vorhergehenden Ansprüche, **dadurch gekennzeichnet, dass** das Dosierventilteilgehäuse (14), die Membran (16) und/oder das Gegenstück (15) zumindest teilweise mit einer hydrophoben oder hydrophilen Beschichtung versehen sind und/oder aus einem hydrophoben oder hydrophilen Material gefertigt sind.

9. Vorrichtung gemäß einem der Ansprüche 5 bis 8, **dadurch gekennzeichnet, dass** das Dosierventilteilgehäuse (14) auf seiner, dem Zellsammler (13) zugewandten Seite, mittig einen Ventilkegel als Gegenstück (15) aufweist sowie 1 bis 30 Bohrungen (19) für das Dosiermedium, die außerhalb einer sichelartigen Nut (20) zur Verankerung der Zellsammelstruktur (18) angeordnet sind.

10. Vorrichtung gemäß einem der Ansprüche 5 bis 9, **dadurch gekennzeichnet, dass** das Dosierventilteilgehäuse (14) und/oder der Zellsammler (13) modular austauschbar sind.

11. Vorrichtung gemäß einem der vorhergehenden Ansprüche, **dadurch gekennzeichnet, dass** das zellsammlermodul (23) zumindest teilweise mit mindestens einem Material beschichtet ist, insbesondere nichtadhäsiven Beschichtungen, enthaltend oder bestehend aus Polyethylenglycol (PEG), Poly(2-hydroxyethyl-methyl(meth)acrylat) (Poly(HEMA)), Silikon, Polymethyl(meth)acylat (PMMA), Polyacrylamid, biokombatiblen Beschichtungen, insbesondere Laminin, Fibronectin, CollagenI/III/IV, Lysin, behandeltem Polystyrol, und/oder eine Plasmabehandlung des Zellsammlers (13) zumindest teilweise erfolgt ist.

12. Vorrichtung gemäß einem der vorhergehenden Ansprüche, **dadurch gekennzeichnet, dass** der Grundkörper (1) mindestens eine Sicherung (22) zur Verhinderung eines Verdrehens des Zellsammlermoduls (23) Im Grundkörper (1) aufweist.

13. Verfahren zur Dosierung von Zellen mittels einer Vorrichtung gemäß einem der Ansprüche 1 bis 12, **dadurch gekennzeichnet, dass** die Trägerflüssigkeit mit den zu dosierenden Zellen durch den Einlasskanal (2) für die Trägerflüssigkeit in Richtung Zellsammler (13) fließt, wobei mittels der Zellerkennungsvorrichtung (6) die Zellzahl ermittelt wird, sich die Zellen im Zellsammlermodul (23) ansammeln und zumindest ein Teil der Trägerflüssigkeit durch den Auslasskanal (3) für die Trägerflüssigkeit abfließt, während das Ventil (10) im Auslasskanal für die Trägerflüssigkeit geöffnet ist und das Ventil (11) im Auslasskanal für die Reinigung geschlossen ist und,
nach Erreichen der zu dosierenden Zellzahl, die Steuerung bewirkt, dass ein Impuls auf den Einlasskanal (5) des Dosiermediums gegeben wird und davor oder gleichzeitig das Ventil (10) im Auslasskanal für die Trägerflüssigkeit (3) geschlossen wird sowie die Diffusorstruktur (9) als passives Ventil im Einlasskanal für die Trägerflüssigkeit (2) wirkt, wobei das Ventil (11) im Auslasskanal für die Reinigungsflüssigkeit (4) geschlossen bleibt, durch den Einlasskanal (5) für das Dosiermedium das Dosiermedium hinzugefügt wird und mindestens eine Zelle in einem Tropfen abgegeben wird.

14. Verfahren gemäß dem vorhergehenden Anspruch, **dadurch gekennzeichnet, dass** durch den Einlasskanal (7) für den Mantelstrom ein weiteres Medium zugegeben wird, das einer Vereinzelung der Zellen dient, und eine Anordnung der Zellen hintereinander im Einlasskanal (2) für die Trägerflüssigkeit bewirkt.

15. Verfahren gemäß einem der Ansprüche 13 oder 14, **dadurch gekennzeichnet, dass** im Anschluss und/oder während des Dosiervorgangs, sofern die Zellzahl überschritten wurde, ein Reinigungsschritt durchgeführt wird, wobei durch den Einlasskanal (5) für das Dosiermedium Reinigungsflüssigkeit eingeleitet wird, und das Ventil (10) des Auslasskanals für die Trägerflüssigkeit (3) sowie das Dosierventil (17) geschlossen sind und die Flüssigkeit durch den Auslasskanal (4) für die Reinigungsflüssigkeit abfließt.

16. Verfahren gemäß einem der Ansprüche 13 bis 15, **dadurch gekennzeichnet, dass** als Trägerflüssigkeit Zellkulturmedium, Salzlösungen, gepufferte Lösungen, isotonische Kochsalzlösung, 2(4-(2-Hydroxyethyl)-1-piperazinyl)-ethansulfonsäure (HEPES)-gepufferte Lösungen, Tris(hydroxy-methyl)-aminomethan (TRIS)-gepufferte Lösungen, Natriumcitrat enthaltenden Lösungen, Trypsin enthaltende Lösungen, Ethylendiamintetraessigsäure (EDTA) enthaltende Lösungen, Antibiotika enthaltende Lösungen, Antimycotika enthaltende Lösungen oder Mischungen hiervon eingesetzt werden.

17. Verfahren gemäß einem der Ansprüche 13 bis 16, **dadurch gekennzeichnet, dass** als Dosiermedium Zellkulturmedium, Salzlösungen, isotonische Kochsalzlösung, 2(4-(2-Hydroxyethyl)-1-pipera-zinyl)-ethansulfonsäure (HEPES)-gepufferte Lösungen, Tris(hydroxymethyl)-aminomethan (TRIS)-gepufferte Lösungen, Fluoreszenzmarker enthaltende Lösungen, 3-(4,5-Dimethylthiazol-2-yl)-2,5-diphenyltetrazoliumbromid (MTT) enthaltende Lösungen oder Mischungen hiervon eingesetzt werden.

18. Verfahren gemäß einem der Ansprüche 13 bis 17, **dadurch gekennzeichnet, dass** als Trägerflüssigkeit und als Dosiermedium identische Lösungen eingesetzt werden.

19. Verfahren gemäß einem der Ansprüche 13 bis 18, **dadurch gekennzeichnet, dass** als Trägerflüssigkeit und als Dosiermedium verschiedene Lösungen engesetzt werden.

20. Verwendung der Vorrichtung gemäß einem der Ansprüche 1 bis 12 zur Dosierung von menschlichen Zellen, tierischen Zellen, Prokaryonten, Eukaryonten und/oder pflanzlichen Zellen.

## Claims

1. Device for metering cells consisting of a basic body (1), there being provided, in at least one plane of the basic body (1), at least one inlet-(2) and outlet channel (3) for the carrier liquid, and also at least one cleaning channel (4) and at least one further inlet channel (5) for the metering medium essentially perpendicular to the channels (2, 3, 4) disposed in this plane, and these channels (2, 3, 4, 5) opening into a cell collector module (23), the cell collector module (23) having a cell outlet (8) with a metering valve (17) and a control unit for the initiation of the metering process and a cell detection device (6) being provided, the cell detection device (6) being disposed on or at the basic body (1) or being integrated in the basic body (1), **characterized in that** the device has at least one inlet channel for a by-pass flow (7) which opens into the at least one inlet channel (2) for the carrier liquid.

2. Device according to claim 1, **characterised in that** the channels (2, 3, 4) have at least respectively one valve (10, 11) and/or one diffuser structure (9).

3. Device according to claim 1 or 2, **characterised in that** the basic body (1) is polygonal, preferably rectangular, and the cell collector module (23) is disposed in the centre.

4. Device according to one of the preceding claims, **characterised in that** the device has at least one inlet channel (7) for a by-pass flow which opens into the at least one inlet channel (2) for the carrier liquid.

5. Device according to one of the preceding claims, **characterised in that** the cell collector module (23) is constructed from a cell collector (13) and a metering valve partial housing (14).

6. Device according to one of the preceding claims, **characterised in that** the metering valve (17) is constructed from a counterpart (15) and a membrane (16).

7. Device according to the preceding claim, **characterised in that** the cell collector (13) has a membrane (16) which is possibly elastic, and the metering valve partial housing (14) has a counterpart (15) which is in particular conical, spherical or cylindrical, the membrane (16) preferably abutting against the counterpart (15) with pre-tension.

8. Device according to one of the two preceding claims, **characterised in that** the metering valve partial housing (14), the membrane (16) and/or the counterpart (15) are provided at least partially with a hydrophobic or hydrophilic coating, and/or are manufactured from a hydrophobic or hydrophilic material.

9. Device according to one of the claims 5 to 8, **characterised in that** the metering valve partial housing (14) has a valve cone as counterpart (15) in the centre on its side orientated towards the cell collector (13) and also 1 to 30 borings (19) for the metering medium which are disposed outside a crescent-like groove (20) for anchoring the cell collector structure (18).

10. Device according to one of the claims 5 to 9, **characterised in that** the metering valve partial housing (14) and/or the cell collector (13) are exchangeable in a modular fashion.

11. Device according to one of the preceding claims, **characterised in that** the cell collector module (23) is coated at least partially with at least one material, in particular non-adhesive coatings comprising or consisting of polyethyleneglycol (PEG), poly(2-hydroxyethylmethyl(meth)acrylate) (poly(HEMA)), silicone, polymethyl(meth)acrylate (PMMA), polyacrylamide, biocompatible coatings, in particular laminin, fibronectin, collagen I/III/IV, lysin, treated polystyrene, and/or a plasma treatment of the cell collector (13) is effected at least partially.

12. Device according to one of the preceding claims, **characterised in that** the basic body (1) has a safety device (22) for prevention of rotation of the cell collector module (23) in the basic body (1).

13. Method for metering cells by means of a device according to one of the claims 1 to 12, **characterised in that** the carrier liquid with the cells to be metered flows through the inlet channel (2) for the carrier liquid in the direction of the cell collector (13), the number of cells being determined by means of the cell detection device (6), the cells accumulating in the cell collector module (23) and at least a part of the carrier liquid discharging through the outlet channel (3) for the carrier liquid, whilst the valve (10) in the outlet channel for the carrier liquid is opened and the valve (11) in the outlet channel for the cleaning is closed and, after achieving the number of cells to be metered, the control unit has the effect that a pulse is passed to the inlet channel (5) of the metering medium and, before or simultaneously, the valve (10) in the outlet channel for the carrier liquid (3) is closed and also the diffuser structure (9) acts as passive valve in the inlet channel for the carrier liquid (2), the valve (11) in the outlet channel for the cleaning liquid (4) remaining closed, the metering medium being added through the inlet channel (5) for the metering medium and at least one cell being discharged in a drop.

14. Method according to the preceding claim, **characterised in that** a further medium which serves for separating the cells is added through the inlet channel (7) for the by-pass flow and effects an arrangement of the cells in succession in the inlet channel (2) for the carrier liquid.

15. Method according to one of the claims 13 or 14, **characterised in that**, subsequently and/or during the metering process, provided that the number of cells has been exceeded, a cleaning step is implemented, cleaning liquid being introduced through the inlet channel (5) for the metering medium and the valve (10) of the outlet channel for the carrier liquid (3) and also the metering valve (17) being closed and the liquid discharging through the outlet channel (4) for the cleaning liquid.

16. Method according to one of the claims 13 to 15, **characterised in that** there are used as carrier liquid, cell culture medium, salt solutions, buffered solutions, isotonic common salt solution, 2(4-(2-hydroxyethyl)-1-piperazinyl)-ethanesulphonic acid (HEPES)-buffered solutions, tris(hydroxymethyl)-aminomethane (TRIS)-buffered solutions, solutions containing sodium citrate, solutions containing trypsin, solutions containing ethylenediaminetetraacetic acid (EDTA), solutions containing antibiotics, solutions containing antimycotics or mixtures hereof.

17. Method according to one of the claims 13 to 16, **characterised in that** there are used as metering medium, cell culture medium, salt solutions, isotonic common salt solution, 2(4-(2-hydroxyethyl)-1-piperazinyl)-ethanesulphonic acid (HEPES)-buffered solutions, tris(hydroxymethyl)-aminomethane (TRIS)-buffered solutions, solutions containing fluorescence markers, solutions containing 3-(4,5-dimethylthiazol-2-yl)-2,5-diphenyltetrazoliumbromide (MTT) or mixtures hereof.

18. Method according to one of the claims 13 to 17, **characterised in that** identical solutions are used as carrier liquid and as metering medium.

19. Method according to one of the claims 13 to 18, **characterised in that** different solutions are used as carrier liquid and as metering medium.

20. Use of the device according to one of the claims 1 to 12 for metering human cells, animal cells, procaryotes, eucaryotes and/or plant cells.

## Revendications

1. Dispositif pour le dosage de cellules, consistant en un corps de base (1), dans lequel, dans au moins un plan du corps de base (1), au moins un conduit d'admission (2) et un conduit de sortie (3) pour le liquide porteur, ainsi qu'au moins un conduit de purification (4) et, pour l'essentiel perpendiculairement aux conduits (2, 3, 4) agencés dans ce plan, au moins un canal d'admission (5) supplémentaire, est prévu pour le milieu de dosage, et ces conduits (2, 3, 4, 5) débouchent dans un module collecteur de cellules (23),
dans lequel le module collecteur de cellules (23) présente une sortie de cellules (8) comportant une valve doseuse (17), et
dans lequel une commande est prévue pour le déclenchement du processus de dosage et un dispositif de reconnaissance de cellules (6), le dispositif de reconnaissance de cellules (6) étant agencé sur ou contre le corps de base (1) ou intégré dans le corps de base (1), **caractérisé en ce que** le dispositif présente au moins un conduit d'admission pour un courant de gaine (7), qui débouche dans le au moins un conduit d'admission (2) pour le liquide porteur.

2. Dispositif selon la revendication 1, **caractérisé en ce que** les conduits (2, 3, 4) présentent respectivement au moins une valve (10, 11) et/ou une structure formant diffuseur (9).

3. Dispositif selon la revendication 1 ou 2, **caractérisé en ce que** le corps de base (1) est polygonal, de préférence rectangulaire, et le module collecteur de cellules (23) est agencé en position centrale.

4. Dispositif selon l'une des revendications précédentes, **caractérisé en ce que** le dispositif présente au moins un conduit d'admission (7) pour un courant de gaine, qui débouche dans le au moins un conduit d'admission (2) pour le liquide porteur.

5. Dispositif selon l'une des revendications précédentes, **caractérisé en ce que** le module collecteur de cellules (23) résulte de l'assemblage d'un collecteur de cellules (13) et d'une partie de boîtier (14) de valve doseuse.

6. Dispositif selon l'une des revendications précédentes, **caractérisé en ce que** la valve doseuse (17) résulte de l'assemblage d'une contrepièce (15) et d'une membrane (16).

7. Dispositif selon la revendication précédente, **caractérisé en ce que** le collecteur de cellules (13) présente une membrane (16), qui est éventuellement élastique, et la partie de boîtier (14) de la valve doseuse présente une contrepièce (15), qui est en particulier conique, sphérique ou cylindrique, la membrane (16) s'appuyant de préférence contre la contrepièce (15) avec une pré-tension.

8. Dispositif selon l'une des deux revendications précédentes, **caractérisé en ce que** la partie de boîtier (14) de la valve doseuse, la membrane (16) et/ou la contrepièce (15) sont au moins partiellement pourvus d'un revêtement hydrophobe ou hydrophile, et/ou sont fabriqués dans un matériau hydrophobe ou hydrophile.

9. Dispositif selon l'une des revendications 5 à 8, **caractérisé en ce que** la partie de boîtier (14) de la valve doseuse présente, sur sa face dirigée vers le collecteur de cellules (13), et en position centrale un cône de valve servant de contrepièce (15), ainsi que 1 à 30 alésages (19) pour le milieu de dosage, qui sont disposés à l'extérieur d'une rainure (20) en forme de faucille, pour permettre un ancrage de la structure collectrice de cellules (18).

10. Dispositif selon l'une des revendications 5 à 9, **caractérisé en ce que** la partie de boîtier (14) de la valve doseuse et/ou le collecteur de cellules (13) sont interchangeables d'une manière modulaire.

11. Dispositif selon l'une des revendications précédentes, **caractérisé en ce que** le module collecteur de cellules (23) est au moins partiellement revêtu d'au moins un matériau, en particulier de revêtements non adhésifs, contenant, ou étant constitués, du polyéthylène glycol (PEG), du poly(méth)acrylate de 2-hydroxyéthylméthyle (poly(HEMA), du silicone, du poly(méth)acrylate de méthyle (PMMA), du polyacrylamide, de revêtements biocompatibles, en particulier de laminine, de fibronectine, de collagène I/III/IV, de lysine, de polystyrène traité, et/ou un traitement plasma du collecteur de cellules (13) est réalisé au moins partiellement.

12. Dispositif selon l'une des revendications précédentes, **caractérisé en ce que** le corps de base (1) présente au moins une sécurité (22) pour empêcher une torsion du module collecteur de cellules (23) dans le corps de base (1).

13. Procédé de dosage de cellules au moyen d'un dispositif selon l'une des revendications 1 à 12, **caractérisé en ce que** le liquide porteur avec les cellules à doser s'écoule par le conduit d'admission (2) pour le liquide porteur en direction du collecteur de cellules (13), procédé dans lequel le nombre de cellules est déterminé au moyen du dispositif de reconnaissance des cellules (6), les cellules s'accumulent dans le module collecteur de cellules (23), et au moins une partie du liquide porteur s'échappe par le conduit de sortie (3) pour le liquide porteur, tandis que la valve (10) est ouverte dans le conduit de sortie pour le liquide porteur, et la valve (11) est fermée dans le conduit de sortie pour la purification, et
après qu'a été atteint le nombre de cellules à doser, la commande fait en sorte qu'une impulsion soit envoyée sur le conduit d'admission (5) du milieu de dosage, et, auparavant ou simultanément, la valve (10) est fermée dans le conduit de sortie pour le liquide porteur (3), et la structure formant diffuseur (9) agit en tant que valve passive dans le conduit d'admission pour le liquide porteur (2), dans lequel la valve (11) reste fermée dans le conduit de sortie pour le liquide de purification (4), le milieu de dosage est ajouté par le conduit d'admission (5) pour le milieu de dosage, et au moins une cellule est dégagée dans une goutte.

14. Procédé selon la revendication précédente, **caractérisé en ce qu'**un milieu supplémentaire est ajouté par le conduit d'admission (7) pour le courant de gaine, milieu qui sert à une individualisation des cellules et provoque un agencement des cellules les unes derrière les autres dans le conduit d'admission (2) pour le liquide porteur.

15. Procédé selon l'une des revendications 13 ou 14, **caractérisé en ce que**, à la suite de et/ou pendant le processus de dosage, et à partir du moment où le nombre des cellules a été dépassé, on procède à une étape de purification, dans laquelle un liquide de purification est mené par le conduit d'admission (5) pour le milieu de dosage, et la valve (10) du conduit de sortie pour le liquide porteur (3), ainsi que la valve doseuse (17), sont fermées, et le liquide s'écoule par le conduit de sortie (4) pour le liquide de purification.

16. Procédé selon l'une des revendications 13 à 15, **caractérisé en ce qu'**on utilise en tant que liquide porteur un milieu de culture cellulaire, des solutions salées, des solutions tamponnées, des solutions isotoniques de chlorure de sodium, des solutions tamponnées par de l'acide 2-(4-(2-hydroxyéthyle)-1-pipérazinyle)-éthanesulfonique (HEPES), des solutions tamponnées par du tris(hydroxyméthyle)-aminométhane (TRIS), des solutions contenant du citrate de sodium, des solutions contenant de la trypsine, des solutions contenant de l'acide éthylène diamine tétraacétique (EDTA), des solutions contenant des antibiotiques, des solutions contenant des antimycotiques ou des mélanges de ceux-ci.

17. Procédé selon l'une des revendications 13 à 16, **caractérisé en ce qu'**on utilise en tant que milieu de dosage un milieu de culture cellulaire, des solutions salées, des solutions isotoniques de chlorure de sodium, des solutions tamponnées par de l'acide 2-(4-(2-hydroxyéthyle)-1-pipérazinyle)-éthanesulfonique (HEPES), des solutions tamponnées par du tris(hydroxyméthyle)-aminométhane (TRIS), des solutions contenant des marqueurs fluorescents, des solutions contenant du bromure de 3-(4,5-diméthylthiazole-2-yl)-2,5-diphényltétrazolium (MTT) ou des mélanges de ceux-ci.

18. Procédé selon l'une des revendications 13 à 17, **caractérisé en ce qu'**en tant que liquide porteur et milieu de dosage, on utilise des solutions identiques.

19. Procédé selon l'une des revendications 13 à 18, **caractérisé en ce qu'**en tant que liquide porteur et milieu de dosage, on utilise des solutions différentes.

20. Utilisation du dispositif selon l'une des revendications 1 à 12 pour le dosage de cellules humaines, de cellules animales, de procaryotes, d'eucaryotes et/ou de cellules végétales.
